# EUROPEAN PATENT SPECIFICATION

(11) **EP 3 808 278 B1**
(45) Date of publication and mention of the grant of the patent: **12.11.2025**
(21) Application number: 19815437.9
(22) Date of filing: 05.06.2019
(51) Int. Cl.: A61B 8/06, A61B 8/12, A61B 8/00, A61B 8/08

(54) **BLOOD FLOW VOLUME MEASUREMENT SYSTEM**
BLUTFLUSSVOLUMENMESSSYSTEM
SYSTÈME DE MESURE DE VOLUME DE FLUX SANGUIN

(30) Priority: 05.06.2018 JP 2018107853; 07.03.2019 JP 2019041155
(43) Date of publication of application: 21.04.2021
(73) Proprietor: Tani, Kazuo, Osaka-shi,Osaka 541-0041 (JP)
(72) Inventor: Tani, Kazuo, Osaka-shi,Osaka 541-0041 (JP)
(74) Representative: McWilliams, David John
(86) International application number: PCT/JP2019/022304
(87) International publication number: WO 2019/235517

(56) References cited:
- JP-A- 2003 275 207
- JP-A- 2003 532 478
- JP-A- 2006 170 751
- JP-A- H0 316 562
- US-A1- 2002 198 459
- US-A1- 2006 106 310
- US-A1- 2007 123 779
- US-A1- 2017 251 930
- US-B1- 6 398 734

## Description

### [TECHNICAL FIELD]

The present disclosure relates to a blood flow rate measurement system for measuring blood flow rates of a blood vessel of a subject such as a human body.

### [BACKGROUND]

For example, measuring blood flow rates in blood vessels of patients (subjects) is necessary before, during, and after an operation, such as a cardiac operation. Thus, blood flow rates can be measured by a blood flow rate probe. The measurement results may then be displayed to doctors at a time of need, such as during an operation. Japanese Patent No. 2915343 discloses a measurement device for such measurement and display.
US2017251930A1 relates to a biological information measurement apparatus including an irradiation unit configured to irradiate a living body with light or sound waves as measurement waves; a detection unit configured to detect measurement waves having passed through the inside of the living body; and a computational unit configured to obtain a change over time in blood flow rate and a change over time in blood vessel cross-sectional area based on a detection result from the detection unit.
US2007123779A1 relates to a technique for computing or monitoring blood viscosity, including measuring a cross sectional area of a arterial segment and a volumetric flow rate of blood flowing through the arterial segment at two or more locations.
US2006106310A1 relates to an ultrasonic monitor implemented on a PCB including a gel pad comprised of a gel layer and a membrane layer. Ultrasonic signals are transmitted between the ultrasonic monitor and a living subject through the gel pad.

### [SUMMARY OF THE INVENTION]

However, the blood flow rate probe is connected to a display device by a cable for transmitting signals. The blood flow rate probe is held along the blood vessels of the patients. The display device is located in an operation room. Thus, the cables extending between the blood flow rate probe and the display device can become disorganized on the floor of the operation room. This can prevent efficient operation procedures.

Therefore, the blood flow rate measurement system needs to be improved. According to the present invention, there is defined a blood flow rate measurement system as defined in claim 1.

In the first aspect of the present disclosure, a blood flow rate measurement system has a blood flow rate probe and a display device. The blood flow rate probe is used to measure a blood flow rate of a blood vessel of the test body and convert it to an electric signal. The display device is used to display the blood flow rate measured by the blood flow rate probe. The blood flow rate data is transmitted from the blood flow rate probe to the display device via wireless communication.

Accordingly, the blood flow rate data is transmitted from the blood flow rate probe to the display device via wireless communication. Thus, a signal transmission cord is unnecessary to transmit the signal from the blood flow rate probe to the display device. Thus, the signal transmission cable does not become disorganized on the floor of, for example, an operation room or a laboratory.

The blood flow rate probe has a monitor to display a part of the blood flow data.

Accordingly, the part of the blood flow data is displayed on the monitor of the blood flow rate probe. Thus, doctors can check, for example, summaries of the blood flow rate, electrocardiogram data, or their changes easily via the monitor of the blood flow rate probe, at their hands without needing to look at the display device. As a result, the doctors have no need to watch the display device to check the blood flow rate frequently, and can further concentrate on the operation.

The blood flow rate probe may be embedded and fixed in the test body for a certain period of time.

### [BRIEF DESCRIPTION OF THE DRAWINGS]

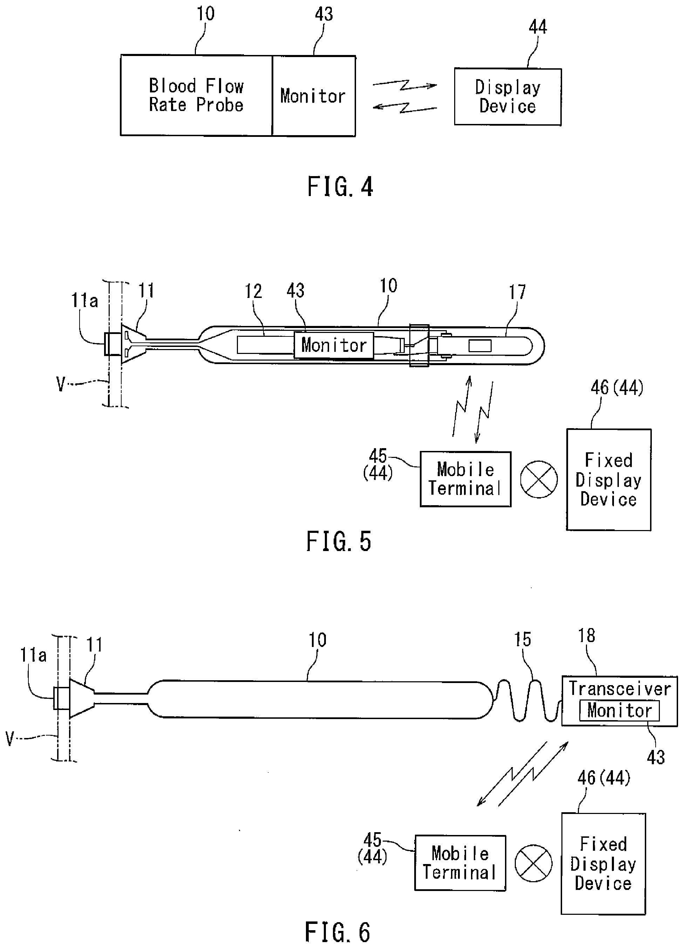

The invention is associated with fig. 4 and the related description. All other embodiments are not part of the current invention but serve as explanation.
[FIG. 1] FIG. 1 is a schematic view of a system configuration of the first embodiment of the present disclosure.
[FIG. 2] FIG. 2 is a system configuration diagram illustrating the first specific example of the first embodiment.
[FIG. 3] FIG. 3 is a system configuration diagram illustrating the second specific example of the first embodiment.
[FIG. 4] FIG. 4 is a schematic view of a system configuration of the second embodiment of the present disclosure.
[FIG. 5] FIG. 5 is a system configuration diagram illustrating the first specific example of the second embodiment.
[FIG. 6] FIG. 6 is a system configuration diagram illustrating the second specific example of the second embodiment.
[FIG. 7] FIG. 7 is a schematic view of a system configuration of the third embodiment of the present disclosure.
[FIG. 8] FIG. 8 is a system configuration diagram illustrating the first specific example of the third embodiment.
[FIG. 9] FIG. 9 is an enlarged perspective view illustrating a major portion of a blood flow rate probe of the second specific example of the third embodiment.
[FIG. 10] FIG. 10 is an enlarged perspective view similar to Figure 9 and illustrates the blood flow rate probe attached to a blood vessel.
[FIG. 11] FIG. 11 is a schematic view of a system configuration of the fourth embodiment of the present disclosure.
[FIG. 12] FIG. 12 is a schematic view of a system configuration of the fifth embodiment of the present disclosure.
[FIG. 13] FIG. 13 is a side view of the blood flow rate probe of the fifth embodiment.
[FIG. 14] FIG. 14 is a perspective view of the blood flow rate probe shown in Figure 13.
[FIG. 15] FIG. 15 is an operation explanatory view of the blood flow rate probe shown in Figure 13.
[FIG. 16] FIG. 16 is a side view of the blood flow rate probe of another example of the fifth embodiment.
[FIG. 17] FIG. 17 is a perspective view of the blood flow rate probe shown in Figure 16.
[FIG. 18] FIG. 18 is a schematic view of a system configuration of the sixth embodiment of the present disclosure.

### [MODES FOR CARRYING OUT THE INVENTION]

### [Configuration of the first embodiment]

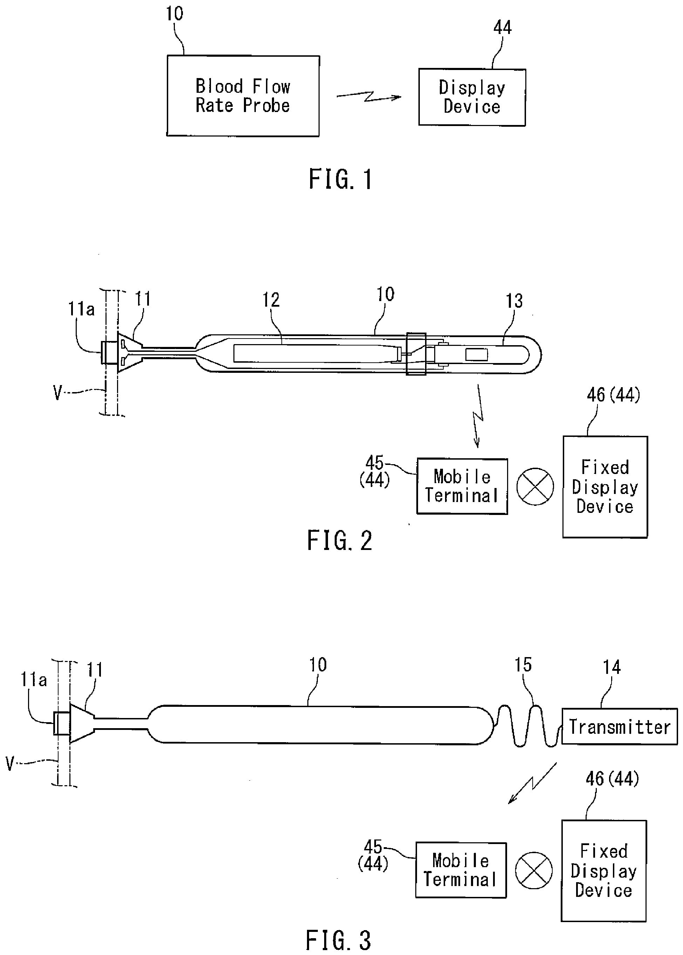

Figure 1 illustrates the first embodiment of the present disclosure. In the first embodiment, a blood flow rate probe 10 and a display device 44 are included. The blood flow rate probe 10 is used to measure blood flow rates of a blood vessel of a patient (test body) during an operation. The blood flow rate probe 10 then converts the measured blood flow rate to electric signals. The display device 44 may display the measured blood flow rate obtained from the blood flow rate probe 10 to doctors during an operation. The blood flow rate data transmission from the blood flow rate probe 10 to the display device 44 can be performed via wireless communication. Thus, the blood flow rate probe 10 may have a transmitter, and the display device 44 may have a receiver. The transmitter and the receiver are not shown in Figure 1. In this case, the wireless communication is performed in the operating room. Accordingly, short range communication, such as Bluetooth^{®}, may be adopted as the means of wireless communication.

### [Configuration of the first specific example of the first embodiment]

Figure 2 illustrates the first specific example of the first embodiment. The blood flow rate probe 10 may adopts the TTFM (Transit Time Flow Measurement) disclosed in Japanese Patent No. 2915343. The blood flow rate probe 10 has a head portion 11 and a reflection plate 11a at an edge portion of the probe 10. The blood flow rate can be measured by placing the blood vessel V of a patient between the head portion 11 and the reflection plate 11a. In this embodiment, the blood flow rate probe 10 is shown as a perspective diagram. The blood flow rate probe 10 has a transmitter 13 for short range communication built in an edge portion, at an end opposite of the head portion 11 of the blood flow rate probe 10. The blood flow rate probe 10 has a battery 12, which supplies electricity to the blood flow rate probe 10, built in a position on the head portion 11 side from the transmitter 13. Thus, obtained blood flow rate data of the blood vessel of the patient is transmitted from the transmitter 13 of the blood flow rate probe 10.

As illustrated in Figure 2, a mobile terminal 45, such as mobile phone or a tablet terminal, may be used as the display device 44 in the first specific example. The mobile terminal 45 has a built-in transceiver (not shown), allowing for short range communication as a standard function. Thus, the mobile terminal 45 can receive the blood flow rate data from the transmitter 13 and display the blood flow rate data on the screen of the mobile terminal 45.

A fixed display device 46 may be used as a display device 44, in addition to the mobile terminal 45. The fixed display device 46 can share the blood flow rate information with the mobile terminal 45, for instance via wireless LAN. The fixed display device 46 may display, for example, an electrocardiogram, a body temperature, a blood pressure, and/or a heart rate, which are measured separately, together with the blood flow rate. Both of the mobile terminal 45 and the fixed display device 46 may not necessarily be provided together as the display device 44. Only either the mobile terminal 45 or the fixed display device 46 may be provided. A plurality of the mobile terminals 45 and/or fixed display device 46 may be installed at different places and may intercommunicate with each other via wireless LAN. Further, the transmitter 13 may have a function to communicate with other devices via wireless LAN. In this case, the transmitter 13 may directly communicate with the fixed display device 46 via wireless LAN, without the need for the mobile terminal 45.

### [Configuration of the second specific example in the first embodiment]

Figure 3 illustrates the second specific example of the first embodiment. Compared to the first specific example, while the second specific example is also characterized in that the blood flow rate probe 10 adopts the TTFM disclosed in Japanese Patent No. 2915343, the blood flow rate probe 10 is a commonly used probe having no transmitter 13. Instead, the output of the blood flow rate probe 10 may be connected to a transmitter 14 via a cord 15. The other components of the second specific example are substantially the same as those of the first specific example in the first embodiment. Accordingly, the same symbols are attached to the same elements. Thus, the repetitive explanation for the same elements are omitted.

The transmitter 14 may be fixed to an operating table. The cord 15 has a length just enough to connect the transmitter 14 fixed to the operating table with the blood flow rate probe 10. Thus, the cord 15 does not become disorganized on the floor of the operating room. The transmitter 14 may be used for short range communication similar to the transmitter 13. Thus, short range communication with the mobile terminal 45 is also possible for the transmitter 14 in addition to the first specific example.

### [Function and effect of the first embodiment]

Signal transmission from the blood flow rate probe 10 to the display device 44 is performed via wireless communication. Thus, a signal transmission cable is unnecessary to transmit signals from the blood flow rate probe 10 to the display device 44. Thus, the signal transmission cable does not become disorganized on the floor of the operating room. When the display device 44 is the mobile terminal 45, doctors or ancillary staffs can easily see the mobile terminal 45 during an operation, so that the doctors and/or ancillary staffs can monitor the blood flow rate easily. The fixed display device 46 may also be used to display the blood flow rate as necessary.

### [Configuration of the second embodiment]

Figure 4 illustrates the second embodiment of the present disclosure. Compared to the first embodiment, the second embodiment is characterized in that the blood flow rate probe 10 has a monitor 43 to display a part of the data showing the blood flow rate. For display on the monitor 43, the blood flow rate probe 10 has a transceiver to transmit and receive data. Further, the display device 44 also has a transceiver to transmit and receive data. The other respects of the second embodiment are substantially the same as those of the first embodiment. Additionally, the same symbols are attached to the same elements. Thus, repetitive explanations for the same elements are omitted.

The monitor 43 receives a part of the contents displayed on the display device 44. The monitor 43 receives the contents from the display device 44 via the transceiver of the display device 44 and the transceiver of the blood flow rate probe 10. After receiving the contents, the monitor 43 displays it.

### [Configuration of the first specific example in the second embodiment]

Figure 5 illustrates the first specific example of the second embodiment. The blood flow rate probe 10 of this example is basically the same as the blood flow rate probe 10 of the first example of the first embodiment. However, a liquid crystal monitor 43 is attached at a position of the blood flow rate probe 10 where the doctors can easily see the monitor 43 of the blood flow rate probe 10 when operating. Additionally, the transmitter 13 is replaced with a transceiver 17 capable of transmitting and receiving. Configurations and functions of the mobile terminal 45 and the fixed display device 46 are the same as those described in the first example of the first embodiment.

The monitor 43 receives, from a transceiver built into the mobile terminal 45, a part of contents displayed on the mobile terminal 45. The monitor 43 receives the contents via the transceiver 17 of the blood flow rate probe 10. Then, the monitor 43 displays the part of this contents.

### [Configuration of the second specific example in the second embodiment]

Figure 6 illustrates the second specific example of the second embodiment. Compared to the second specific example in the first embodiment, the transmitter 14 is replaced with a transceiver 18 in this example. However, in other respects, both may be substantially the same.

In this example, the transceiver 18 has the monitor 43, similar to the first specific example of the second embodiment (e.g., see Figure 5). The monitor 43 receives a part of the contents displayed on the mobile terminal 45 from the mobile terminal 45, via the transceiver18, and then displays the same.

### [Function and effect of the second embodiment]

The blood flow rate probe 10 has the monitor 43 configured to display a part of the data corresponding to the blood flow rate. Thus, doctors can check, for example, summaries of the blood flow rate, electrocardiogram data, or any changes thereof on the monitor 43 at hand during an operation. That is, the doctors can check those data easily via the monitor 43 of the blood flow rate probe 10, without needing to look at the mobile terminal 45 or the fixed display device 46. As a result, the doctors have no need to frequently look up the mobile terminal 45 or the fixed display device 46 to check the blood flow rate, and thus can concentrate on the operation.

### [Configuration of the third embodiment]

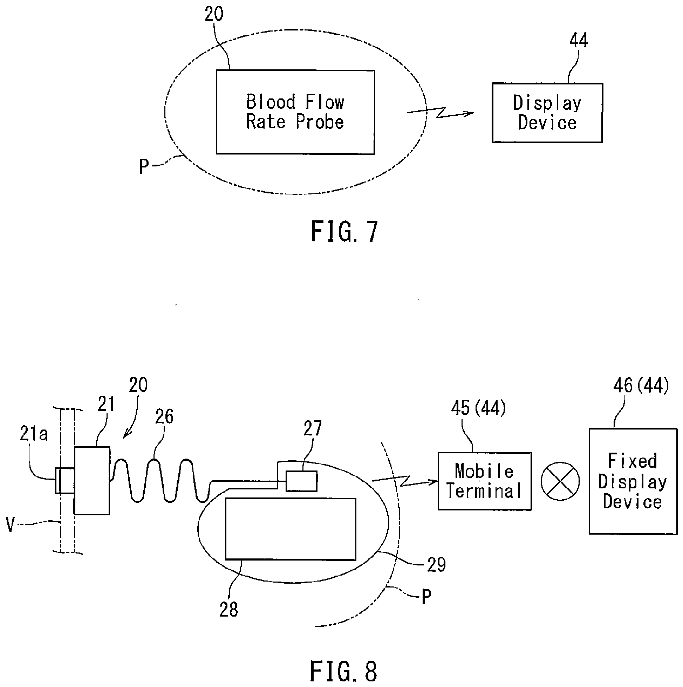

Figure 7 illustrates the third embodiment of the present disclosure. Compared to the first embodiment, the third embodiment is characterized in that the blood flow rate probe 10 is replaced with a blood flow rate probe 20. The blood flow rate probe 20 is configured to be embedded in the test body (e.g., the patient P). The other aspects of the third embodiment are substantially the same as that of the first embodiment. Thus, the repetitive explanations for the same elements are omitted.

### [Configuration of the first specific example in the third embodiment]

Figure 8 illustrates the first specific example of the third embodiment. The blood flow rate probe 20 has a blood flow rate probe body 21, a transmitter 27, and a cord 26. The blood flow rate probe body 21 and the transmitter 27 may be connected electrically by the cord 26. The blood flow rate probe 20 has basic functions which are the same as those of the blood flow rate probe 10 of the first embodiment (e.g., see Figure 2). The blood flow rate probe body 21 adopts the TTFM. The blood vessel V located in the patient P is placed between the probe body 21 and a reflection plate 21a to measure the blood flow rate. Additionally, the transmitter 27 is accommodated in a casing 29, together with a battery 28 that is the power source of the blood flow rate probe 20. The casing 29 may be made of materials, such as polypropylene, silicone, having biocompatibility and allowing radio wave propagation therethrough. Each element of the blood flow rate probe 20 is fixed in the test body, by appropriate methods.

### [Configuration of the second specific example in the third embodiment]

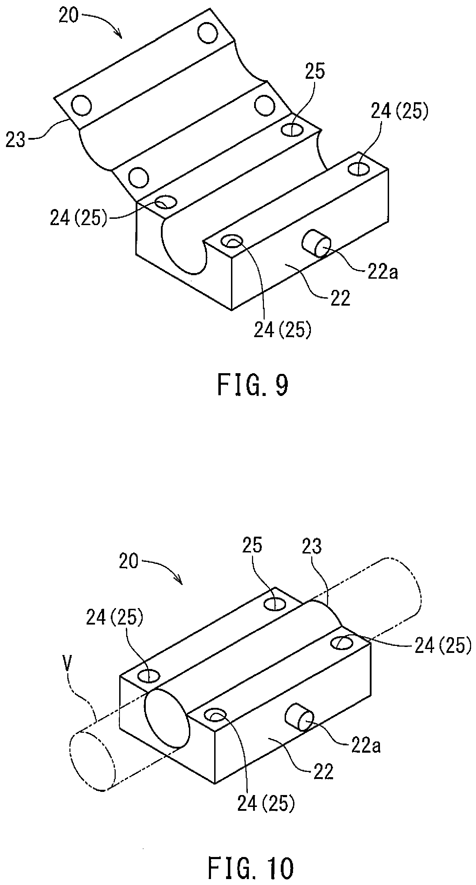

Figures 9 and 10 illustrate the second specific example of the third embodiment. Compared to the first specific example of the third embodiment, the second specific example of the third embodiment is characterized in only that the blood flow rate probe body 21 is replaced with another blood flow rate probe body 22. The other respects in both examples are substantially the same. Thus, the repetitive explanations or illustrations for the same elements are omitted. It is noted that the blood flow rate probe 20 shown in Figures 9 and 10 may also be used in the fourth embodiment described later.

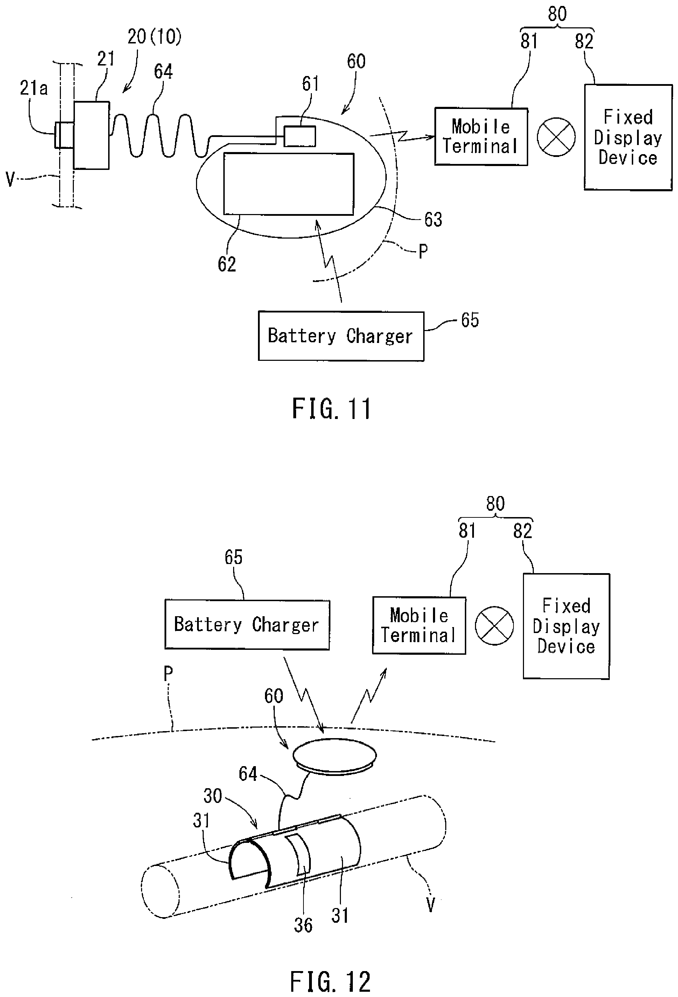

The blood flow rate probe body 22 adopts the TTFM to measure the blood flow rate, similar to that of the blood flow rate probe body 21. The blood flow rate probe body 22 may be covered by a lid 23. This allows the blood vessel V of the patient P to be placed within the blood flow rate probe body 22. Thus, the blood flow rate probe body 22 is configured to measure the blood flow rate. Measurement signals may be sent to the cord 26 (or the cord 64 in Figure 11) via the connecter 22a (see Figures 8 and 11). The lid 23 may be fixed with screws 25 in a state so it covers the blood flow rate probe body 22. Some of the screws 25 can be used as electrodes for electrocardiogram. That is, the blood flow rate probe body 22 can also detect electrocardiograph signals at the same time.

### [Function and effect of the third embodiment]

The blood flow rate and the electrocardiogram can be measured continuously for a certain period of time after an operation by using the blood flow rate probe 20 embedded in the body of the patient P.

### [System configuration in the fourth embodiment]

Figure 11 illustrates a system configuration in alignment with the fourth embodiment of the present disclosure. The fourth embodiment includes the blood flow rate probe 20 and a display device 80. The blood flow rate probe 20 can be used to measure the blood flow rate of the blood vessel V of the patient (test object) P and convert the measurement results into electric signals. The display device 80 can display the measured blood flow rates, so as to be viewable by healthcare professionals, including doctors. The blood flow rate probe 20 is configured to be embedded in the patient P for a certain period during or after an operation. The display device 80 may be placed at a position located outside the patient P where the healthcare professionals including the doctors, can easily see the display device 80.

The blood flow rate data obtained from the blood flow rate probe 20 is transmitted to a first transmitter 61 via a freely bendable cord 64. Then, the blood flow rate data is wirelessly transmitted from the first transmitter 61 to the display device 80. The freely bendable cord corresponds to an electric connecting member in the present disclosure. The first transmitter corresponds to a transmitter in the present disclosure. The distance between the blood flow rate probe 20 and the first transmitter 61 may change. However, the cord 64 connecting the blood flow rate probe 20 and the first transmitter 61 is freely bendable, and thus the cord 64 can flexibly adapt to the change of distance.

The first transmitter 61 is configured to be embedded in the patient P, in a state where the first transmitter 61 is accommodated in a casing 63. The casing 63 accommodates a battery 62, in addition to the first transmitter 61. The battery corresponds to a power source device in the present disclosure. The battery 62 may supply power to the first transmitter 61, and may also supply power to the blood flow rate probe 20, via the cord 64. The battery 62 may be recharged by a non-contact battery recharger 65 disposed outside the body. For example, an electromagnetic induction system or a radio wave receiving system may be used for charging the battery 62. However, a primary battery can be used as the battery 62, instead of the secondary battery, which is a rechargeable one. The first transmitter 61 may wirelessly communicate while still in the operating room. Thus, short distance communications such as Bluetooth^{®}, may be used as the wireless communication method. The casing 63 may be made of, for example, a polypropylene resin or a silicone resin. Furthermore, the blood flow rate probe 20, the cord 64, the casing 63, and the other connecting members may have watertight and airtight structures allowing them to be buried in the body.

The mobile terminal 81, such as a mobile phone or a tablet terminal, may be used as the display device 80. The mobile terminal 81 has a built-in transceiver (not shown), which enables short distance communication as a standard function. Thus, the mobile terminal 81 may receive the blood flow rate data sent by the first transmitter 61 and display the blood flow rate on its own display. The mobile terminal 81 may continuously display the blood flow rate, although the mobile terminal 81 may display the blood flow rate at appropriate timing, such as intermittently.

In addition to the mobile terminal 81, a fixed display device 82 may be used as the display device 80. The fixed display device 82 can share the blood flow rate information with the mobile terminal 81 via wireless LAN. The fixed display device 82 may display, for example, an electrocardiogram, a body temperature, a blood pressure and a heart rate, which are measured separately, in addition to the blood flow rate. The mobile terminal 81 and the fixed display device 82 are not necessarily used at the same time. That is, only one of either of the mobile terminal 81 or the fixed display device 82 may be used. A plurality of the mobile terminals 81 and the fixed display devices 82 may be installed at different places and intercommunicate via wireless LAN.

The blood flow rate probe 20 (10) can be used to measure the blood flow rate using the TTFM, which is publicly known. When the blood flow rate is measured, the blood vessel V of the patient P is placed between the blood flow rate probe body 21 and the reflection plate 21a. However, the method for measuring the blood flow rate is not limited to the TTFM. The blood flow rate probe 20 can be used for other blood flow rate measuring devices adopting different methods.

### [Function and effect of the fourth embodiment]

According to the fourth embodiment, the first transmitter 61 and the battery 62 are connected to the blood flow rate probe 20 (10) via the freely bendable cord 64. Further, the first transmitter 61 and the battery 62 may be separated from the blood flow rate probe 20 (10), so as to be located on the surface side of the patient P. Thus, if the blood vessel V is located in a narrow space in the body, only the blood flow rate probe 20 (10) can be inserted into the narrow space. This allows the first transmitter 61 and the battery 62 to be placed appropriately in a location separated from the blood flow rate probe 20 (10). As mentioned above, the first transmitter 61 and the battery 62 can be located on the surface side of the patient **P,** separated from the blood flow rate probe 20 (10). With this arrangement, a volume of a dielectric body (a portion of the patient P) within the communication path between the first transmitter 61 and the display device 80 can be reduced. This can improve the communication environment. Additionally, the first transmitter 61 and the battery 62 can be easily accessed from the surface side. Thus, maintenance of the first transmitter 61 and the battery 62 can be improved.

Furthermore, the first transmitter 61 and the battery 62 may be accommodated in a single casing 63. This allows the first transmitter 61 and the battery 62 to be handled as a single piece. Additionally, the first transmitter 61 and the battery 62 are covered by the casing 63. Thus, the first transmitter 61 and the battery 62 are protected from being exposed in the body.

### [System configuration in the fifth embodiment]

Figure 12 illustrates a system configuration in alignment with the fifth embodiment of the present disclosure. Compared to the fourth embodiment, the fifth embodiment is characterized in that the blood flow rate probe 20 (10) is replaced by another blood flow rate probe 30. The other respects, in the fourth and the fifth embodiments are substantially the same. Thus, the repetitive explanations or illustrations for the same elements are omitted.

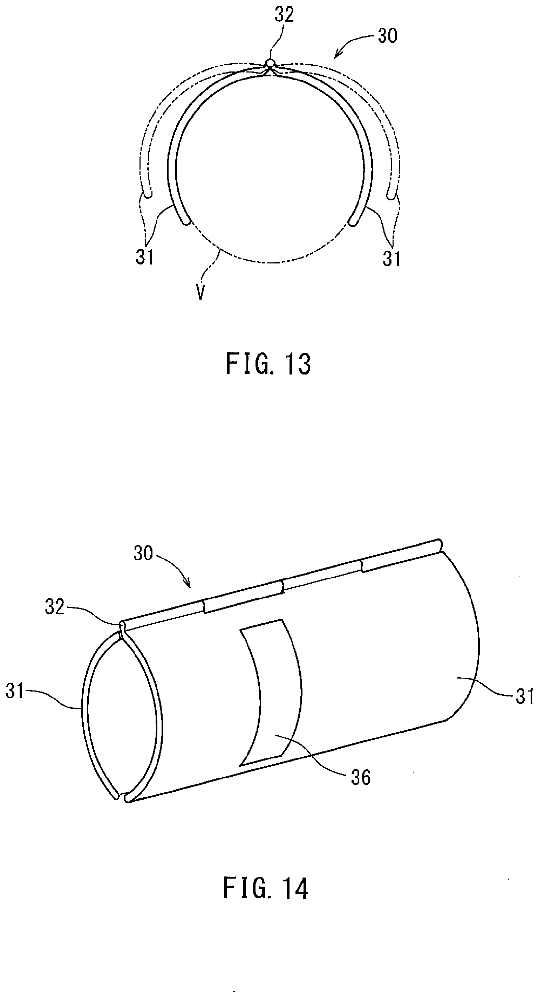

Figures 13 and 14 are enlarged views of the blood flow rate probe 30. The blood flow rate probe 30 has a pair of torso members 31 configured to be openable by a hinge member 32. The pair of the torso members 31 are made of, for example, a polypropylene resin. The pair of the torso members 31 have a curved shape so that the blood vessel V of the patient P can be placed between the torso members 31. When the blood vessel V is placed between the pair of the torso members 31, the torso members 31 are spaced apart from each other, as shown by the imaginary lines in Figure 13. After the blood vessel is inserted between the pair of torso members 31, the torso members 31 are closed toward each other so that the torso members 31 can fit the blood vessel V therebetween, as shown by solid lines in Figure 13. When the blood vessel V is placed between the pair of the torso members 31, as shown by the solid lines in Figure 13, the torso members 31 form a partially opened circular pipe shape that allows the blood vessel V to pass therethrough. With this arrangement, the pair of torso members 31 can wrap around an outer peripheral surface of the blood vessel V.

In this way, the pair of the torso members 31 is configured to be openable about the hinge member 32. Thus, it is easy to place the blood vessel V between the pair of torso members 31. The operator can open the pair of torso members 31 with their fingers, via the hinge member 32. Even if the operator does not touch the torso members 31, a frictional force applied between the pair of the torso members 31 keeps the relative angle between the torso members 31.

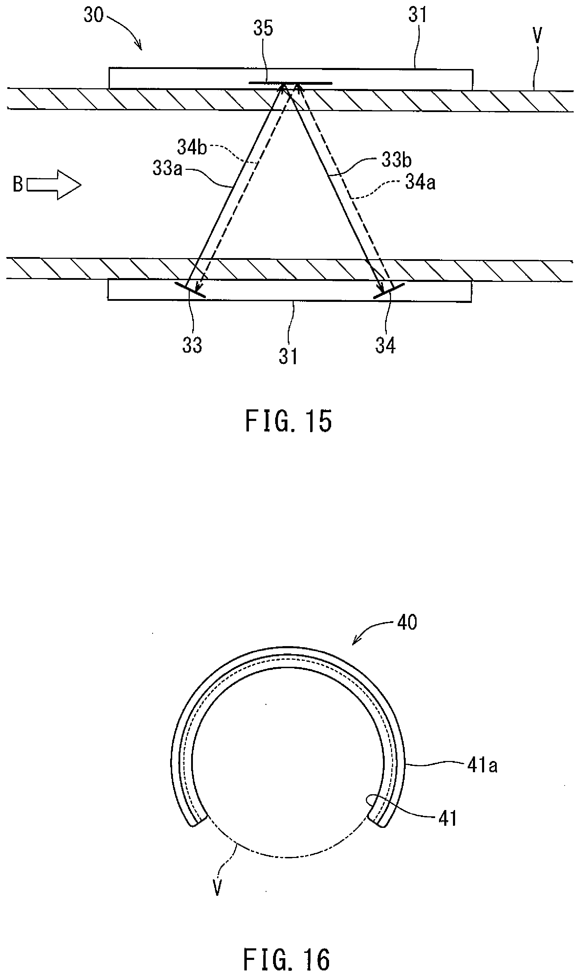

Figure 15 illustrates a principle of measuring the blood flow rate with the blood flow rate probe 30. The blood flow rate probe 30 may adopt the publicly known TTFM to measure the blood flow rate. One torso member 31 has a reflection plate 35 on its inner wall surface. The other torso member 31 has a first transducer 33 and a second transducer 34 on its inner wall surface. The first and the second transducers 33, 34 are opposite to the reflection plate 35. The reflection plate 35 reflects ultrasonic waves 33a, 34a respectively emitted from the first and the second transducers 33, 34 in a state that the blood vessel V is placed between the reflection plate 35 and the first and the second transducers 33, 34. Then, the first and the second transducers 33, 34 receive the reflected ultrasonic waves 34b, 33b, respectively. The torso member 31 has a processing circuit 36 positioned at its outer wall (e.g., see Figure 14). The processing circuit 36 can determine a flow rate of a blood flow B in the blood vessel V based on time lags between transmission and receipt of the ultrasonic waves in the first and the second transducers 33, 34. The measurement results of the blood flow rates by the blood flow rate probe 30 are converted to electric signals. Then, the electric signals are sent to a transmitter unit 60 via the cord 64, as shown in Figure 12.

To measure the blood flow rate with the blood flow rate probe 30, the reflection plate 35 needs to reflect the ultrasonic waves 33a, 34a respectively emitted from the first and the second transducers 33, 34. Additionally, the first and the second transducers 33, 34 need to receive the reflected ultrasonic waves 34b, 33b respectively. Thus, the relative angle between the pair of the torso members 31 needs to be maintained, so that the blood flow rate can be measured. Thus, the blood flow rate probe 30 may have different sizes depending on the diameters of the blood vessel V which is the target to be measured. In this way, the relative angles between the pair of the torso members 31 can be maintained to measure the blood flow rate.

### [Another example of the blood flow rate probe 30]

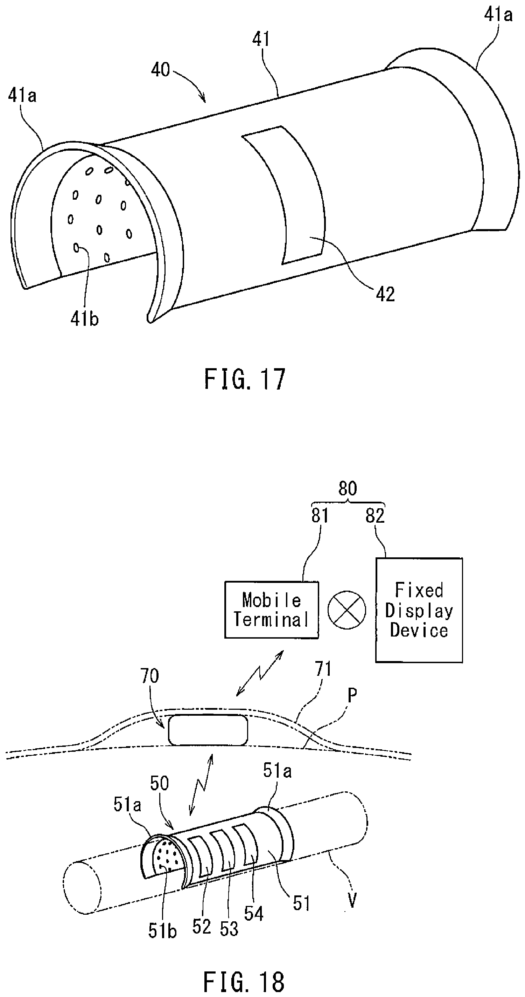

Figures 16 and 17 illustrate another example of the blood flow rate probe 30 in the fifth embodiment. A blood flow rate probe 40 is different from the blood flow rate probe 30. The main difference is that the blood flow rate probe 40 has a torso member 41 having an integrated structure with no hinge member 32. The torso member 41 has diameter-expanded portions 41a at both edges (e.g., see Figure 17). Each of the diameter-expanded portions 41a has a shape that expands gradually toward an edge side of the torso member 41. The torso member 41 may have a number of small projections 41b at its inner wall, which may increase frictional resistance between the inner wall and the blood vessel V. The blood flow rate probe 40 can be used to measure the blood flow rate by the TTFM, similar to the blood flow rate probe 30. The blood flow rate probe 40 may have a processing circuit 42 at its outer wall, similar to the processing circuit 36 of the blood flow rate probe 30.

The blood flow rate probe 40 is not configured to be openable in the same way as the blood flow rate probe 30. Instead, the blood vessel V can be inserted into the torso member 41 by deforming the blood vessel V into an elliptical shape. However, the torso member 41 may also be elastically deformed to slightly widen its opening when the blood vessel V is inserted into the torso member 41. The blood flow rate probe 40 may be prepared in various sizes, depending on the diameters of the blood vessels V to be measured.

The blood flow rate probe 40 has a diameter-expanded portions 41a at each of its edges. The diameter-expanded portions 41a can prevent the both edges of the torso member 41 from digging into the outer peripheral surface of the blood vessel V, for instance when the blood vessel V is inserted into the torso member 41. Furthermore, the torso member 41 has a number of projections 41b at its inner wall. This can prevent the torso member 41 holding the blood vessel V, from slipping along the surface of the blood vessel V, for instance due to the gravity when the blood vessel V is in a vertical or substantially vertical state.

### [Function and effect of the fifth embodiment]

According to the fifth embodiment, the curved shape torso members 31, 41 can wrap around the outer peripheral surface of the blood vessel V for which the blood flow rate is being measured. The torso members 31, 41 can be also attached easily to the blood vessel V located in a narrow space. In this way, the torso members 31, 41 can be used for various blood vessels V.

### [System configuration in the sixth embodiment]

Figure 18 illustrates a system configuration according to the sixth embodiment of the present disclosure. In the fifth embodiment, the torso members 31, 41 send the measured data to a transmitter unit 60 via a cord 64 (e.g., see Figure 12). On the other hand, the sixth embodiment is characterized in that a torso member 51 send measured data to a transmitter unit 70 via wireless communication. The other configurations in the sixth embodiment are substantially the same as the fifth embodiment. Accordingly, the same symbols are attached to the same elements. Thus, repetitive explanations for the same elements are omitted.

The torso member 51 of the blood flow rate probe 50 in the sixth embodiment has a configuration similar to the torso member 41 in the fifth embodiment. The difference between them is only a configuration to connect the transmitter unit 70 with the torso member 51 via wireless communication. The other configurations may be substantially the same. The torso member 51 may also have diameter-expanded portions 51a and projections 51b that are substantially the same as the diameter-expanded portions 41a and the projections 41b of the torso member 41.

The torso member 51 has a power supply equipment 52, a processing circuit 53, and a second transmitter at its outer peripheral surface. The second transmitter corresponds to a transmitter in the present disclosure. The power supply equipment 52 is wirelessly fed power from a power source device (not shown) accommodated in the transmitter unit 70. The fed power supplies working power to the processing circuit 53 and the second transmitter 54. The wireless power feeding may adopt, for example, a feeding method using radio waves. The wireless power feeding corresponds to an electric connection means of the present disclosure. The processing circuit 53 can be used to measure the blood flow rate based on the time lags between transmission and receipt of the ultrasonic waves, for instance as determined by the TTFM used by the processing circuit 42 in the fifth embodiment. The second transmitter 54 may transmit the blood flow rate data determined by the processing circuit 53 to a first transmitter (not shown) accommodated in the transmitter unit 70. The second transmitter 54 may communicate with the first transmitter via in vivo communication. The transmitter unit 70 may be attachable to an outside the surface of the patient P, for instance by a belt 71. The second transmitter 54 may be able to communicate directly with the mobile terminal 81, without using the first transmitter of the transmitter unit 70, depending on certain conditions, such as a position of the blood vessel V in the patient P.

### [Function and effect of the sixth embodiment]

According to the sixth embodiment, the blood flow rate probe 50 embedded in the patient P's body can be used to measure blood flow rate. The measured data is sent from the second transmitter 54 to the display device 80 located outside of the patient P's body via the first transmitter, which is in the transmitter unit 70, by wireless communication. Then, the data is displayed on the display device 80. The power source device, which is provided in the transmitter unit 70, supplies electric power to the blood flow rate probe 50, via the power supply equipment 52. The transmitter unit 70 may be fixed to the outside of the surface of the patient P. Thus, only the blood flow rate probe 50 is embedded in the patient P. This can decrease the physical burden during the measurement of the blood flow rate on the patient P. Thus, the blood flow rate measurement system of the sixth embodiment allows the measurement to continue for a relatively long term, such as several months, after the operation. The torso member 51 of the blood flow rate probe 50 has the power supply equipment 52. The power supply equipment 52 may be mainly used to stabilize the power supply when receiving the electric energy from the power source device of the transmitter unit 70. Thus, the power supply equipment 52 may not need to keep supplying the accumulated electricity for a long time. Accordingly, the size of the blood flow rate probe 50 may only be slightly bigger than a blood flow rate probe having a normal power source device.

### [The other embodiment]

While the present disclosure has been described herein with reference to specific embodiments, various kinds of changes, additions, or deletion may be possible, for example, but are not limited to their appearances or structures. For example, the blood flow rate measurement system of the present disclosure may be applied to not only human blood vessels, but also animal blood vessels. Additionally, the blood flow rate measurement system of the present disclosure may be applied to not only blood vessels of the heart, but also blood vessels of the other organs of the test body. Furthermore, the blood flow rate measurement system of the present disclosure may be applied to, for example, artificial hearts or artificial blood vessels.

## Claims

1. A blood flow rate measurement system, comprising:
a blood flow rate probe (10) configured to measure a blood flow rate of a blood vessel (V) of a test body (P) and to convert the blood flow rate to an electric signal; wherein the blood flow rate probe further comprises a transceiver to transmit and receive data, and
a display device (44) configured to display a blood flow rate measured by the blood flow rate probe (10) together with other data which are measured separately, the display device also comprises a transceiver to transmit and receive data,
wherein the system is configured so that data of blood flow rate is transmittable from the blood flow rate probe (10) to the display device (44) via wireless communication, and
wherein the blood flow rate probe (10) comprises a monitor (43) configured to display a part of data displayed on the display device (44) showing the blood flow rate
wherein the system is configured so that the part of the data showing the blood flow rate is transmitted from the display device (44) to the blood flow rate probe (10) via wireless communication.

## Patentansprüche

1. Blutflussratenmesssystem, umfassend:
eine Blutflussratensonde (10), die dazu konfiguriert ist, eine Blutflussrate eines Blutgefäßes (V) eines Testkörpers (P) zu messen und die Blutflussrate in ein elektrisches Signal umzuwandeln; wobei die Blutflussratensonde weiter einen Transceiver umfasst, um Daten zu übertragen und zu empfangen, und
eine Anzeigevorrichtung (44), die dazu konfiguriert ist, eine von der Blutflussratensonde (10) gemessene Blutflussrate zusammen mit anderen Daten, die separat gemessen werden, anzuzeigen, wobei die Vorrichtung auch einen Transceiver umfasst, um Daten zu übertragen und zu empfangen,
wobei das System so konfiguriert ist, dass Daten der Blutflussrate von der Blutflussratensonde (10) über drahtlose Kommunikation an die Anzeigevorrichtung (44) übertragbar sind, und
wobei die Blutflussratensonde (10) einen Monitor (43) umfasst, der dazu konfiguriert ist, einen Teil der Daten, die auf der Anzeigevorrichtung (44) angezeigt werden, der die Blutflussrate zeigt, anzuzeigen
wobei das System so konfiguriert ist, dass der Teil der Daten, der die Blutflussrate zeigt, von der Anzeigevorrichtung (44) über drahtlose Kommunikation an die Blutflussratensonde (10) übertragen wird.

## Revendications

1. Système de mesure de débit sanguin, comprenant :
une sonde (10) de débit sanguin configurée pour mesurer un débit sanguin d'un vaisseau sanguin (V) d'un corps (P) à l'essai et pour convertir le débit sanguin en un signal électrique ; dans lequel la sonde de débit sanguin comprend en outre un émetteur-récepteur pour transmettre et recevoir des données, et
un dispositif d'affichage (44) configuré pour afficher un débit sanguin mesuré par la sonde (10) de débit sanguin ainsi que d'autres données qui sont mesurées séparément, le dispositif d'affichage comprend également un émetteur-récepteur pour transmettre et recevoir des données,
dans lequel le système est configuré de manière à ce que des données de débit sanguin puissent être transmises depuis la sonde (10) de débit sanguin au dispositif d'affichage (44) par communication sans fil, et
dans lequel la sonde (10) de débit sanguin comprend un moniteur (43) configuré pour afficher une partie des données affichées sur le dispositif d'affichage (44) montrant le débit sanguin
dans lequel le système est configuré de manière à ce que la partie des données montrant le débit sanguin soit transmise depuis le dispositif d'affichage (44) à la sonde (10) de débit sanguin par communication sans fil.
